# EUROPEAN PATENT SPECIFICATION

(11) **EP 1 399 581 B1**
(45) Date of publication and mention of the grant of the patent: **11.07.2007**
(21) Application number: 02764602.5
(22) Date of filing: 24.06.2002
(51) Int. Cl.: C12Q 1/04, C12Q 1/30, G01N 33/02

(54) **DETECTION OF MICROBIOLOGICAL CONTAMINANTS IN SAMPLES OF NON-LIVING MATERIAL**
DETEKTION VON MIKROBIOLOGISCHER KONTAMINIERUNG IN UNBELEBTE MATERIER
PROCEDE DE DETECTION DE CONTAMINANTS CELLULAIRES DANS DES ECHANTILLONS DE MATIERE NON VIVANTE

(30) Priority: 29.06.2001 EP 01115920
(43) Date of publication of application: 24.03.2004
(73) Proprietor: Vivactiss BVBA, 3000 Leuven (BE)
(72) Inventor: VERHAEGEN, Katarina, B-3000 Leuven (BE)
(74) Representative: Bird, Ariane
(86) International application number: PCT/EP2002/007153
(87) International publication number: WO 2003/002748

(56) References cited:
- EP-A- 0 184 260
- EP-A- 0 221 663
- FR-A- 2 611 744
- GB-A- 2 220 066
- US-A- 5 760 406
- US-A- 6 110 661
- SWAIN A ISBN 0-7225-0492-6: "Rapid microbial assay technology." INTERNATIONAL INDUSTRIAL BIOTECHNOLOGY 1988 BIOTECH. CENT., CRANFIELD INST. OF TECH., CRANFIELD MK43 0AL, UK, vol. 8, no. 2, pages 11-15, XP008014341
- PHILIP BABCOCK GROVE: "Webster's third new international dictionary" 1986, MERRIAM-WEBSTER INC , SPRINGFIELD, MASSACHUSETTS, USA * pages 474,1872 *
- TILLBERG ET AL: "Microcalorimetric measurements of the heat evolution and its sensitivity to dinitrophenol during different stages of synchronous cultures of scenedesmus" PLANT PHYSIOL, vol. 48, 1971, pages 779-782,
- ITO ET AL: "Effects of 2,4-dinitrophenol and other metabolic inhibitors on the thermograms of Ehrlich ascites carcinoma cells registered with a microcalorimeter" CANCER RESEARCH, vol. 44, no. 5, 1984, pages 1985-1990,

## Description

### Field of the invention

The present invention relates to a method for detecting cellular contaminants in food (and related) products by interfering with or detection of metabolic reactions.

### Background

The contamination by undesirable pathogens in foodstuffs and water represents a significant threat to public health. Monitoring efforts rely on conventional microbiological techniques to detect the presence of bacteria or other pathogens, typically including the growth of bacteria on nutrient media. Conventional bacterial identification and confirmation techniques utilizing membrane filtration require culturing of a specimen on selective media with selection of potential colony types based on morphology and specific color, etc., to make a presumption, followed by a growth on a non-selective enrichment medium which is then transferred to a carbohydrate and pH indicator panel for confirmation. In many cases, several different media must be employed in order to discriminate one species from another, or to ensure all contaminants are identified.
For certain membrane filtration procedures, the complete process can take several days. This requires storage under adequate conditions of the food from which the sample is taken, entailing additional costs and compromising the freshness of the food product for the consumer.

More rapid systems for the detection of bacteria have been developed based on the determination of hydrolytic enzymes using chromogenic or fluorogenic enzyme substrates and dyes (Hansen, W., Yourassowsky, E., J. Clin. Micro., 20(4):1177-1179, 1984). There have also been attempts to measure the bacterial concentration in food by measuring specific metabolic byproducts of individual microorganisms. Though useful for the identification of specific bacterial species, to ensure coverage of all possible bacterial contaminants, several of these methods need to be combined. More general detection methods include electrical impedance assays, ATP assays, antibody-based assays, or isotopic assays such as carbon 14 substrate assays (WO96/40980). However, these detection methods require significant technical skill and specialized equipment.

Thus there is an urgent need for a fast, simple and general method for the detection of viable pathogens in food products. According to the present invention, viable pathogens are detected by interfering with basic metabolic reactions and measuring the heat released thereby as described in the appended claims.

The enzyme catalase, which degrades hydrogen peroxide into water and oxygen, is present in all animal and plant cells as well as in bacteria. In animal cells it is present in the peroxisomes to counter the potential deleterious effect of the hydrogen peroxide produced as a byproduct in the degradation of fatty acids and amino acids.

All processes involved in growth and metabolism of cells require an input of energy. ATP is the universal currency of energy found in all types of organisms and is produced by proton concentration gradients and electrical potential gradients across membranes, which in turn are powered by the energy absorbed by photosynthesis (in plants) or generated by the oxidation of metabolic products of sugars and fatty acids (mitochondria and aerobic bacteria). In the case of bacteria, the electron transport system is in the cell membrane and a proton gradient is established across the membrane when protons are pumped out of the cell.

Swain et al (1988) disclose a microcalorimetric method to enumerate contaminating microorganisms in food. No uncoupling agent is added in this method.

Brown fat of mammals is a tissue specialized in generating heat. It is characterized by the abundant presence of mitochondria in which an inner-membrane protein (Uncoupling Protein Homologue or UCPH) acts as a natural uncoupler of oxidative phosphorylation, short-circuiting the membrane proton gradient across the mitochondrial membrane and converting the energy released by oxidation of NADH into heat (Cannon & Nedergaard, 1985, Essays in Biochem. 20:110-165; Himms-Hagen J., 1989, Prog. Lipid Res. 28:67-115; Nicholls & Locke, 1984, Physiol. Rev. 64:1-64; Klingenberg M., 1990, Trends Biochem. Sci. 15:108-112; Klaus S. et al., 1991, Int. J. Biochem. 23:791-801). Other molecules have been found to have a similar effect: 2, 4-dinitrophenol (DNP) acts as a membrane transporter for H+, bypassing the ATP synthesis system normally associated with H+. Such molecules abolish the synthesis of ATP and dispense with any requirement for ADP in the oxidation of NADH or in the transport of electrons, so that the energy released by the oxidation of NADH is converted to heat.

However, none of the documents cited above describe or suggest the present invention.

### Summary of the invention:

The present invention relates to a method for detecting the contamination of a sample with living cells, such as bacteria, in non-living material, preferably food products, by stimulating or blocking one or more metabolic reactions in the living cells. Preferably, the metabolic reactions which are targeted are associated with energy production. The amount of associated energy change can be monitored and is a measure of the degree of contamination.

The present invention relates to the detection of living animal or plant cell contaminant in non-living material, by uncoupling, in the contaminant, the electron transport from ATP synthesis and measuring the associated energy release. The energy release is measured by way of microcalorimetric detection, based on heat conduction or radiation.

In a preferred embodiment of the invention, the energy which is generated by uncoupling of the electron transport from ATP synthesis is measured by a microcalorimeter comprising a thermopile. Within the thermally insulated calorimeter, the energy change results in a temperature change of the sample.

According to a preferred embodiment of the invention uncoupling of the electron transport from ATP synthesis in the cellular contaminant is achieved by addition of an uncoupling agent, such as, but not limited to molecules such as dinitrophenol (DNP), which is capable of dissipating the transmembrane proton gradient by acting as a transmembrane proton shuttle. Alternatively, natural molecules such as the uncoupling protein homologue (UCPH or UCP2) or functional equivalents thereof, or (for some embodiments) molecules capable of regulating the activity of UCPH can be applied.

### Description of figures

The following detailed description, given by way of example, but not intended to limit the invention to specific embodiments described, may be understood in conjunction with the accompanying Figure, in which:
- Fig 1.: Effect of Carbonyl cyanide 3-cholorphenylhydrazone on heat production in hepatocytes
- Fig 2.: Effect of sodium azide on catalase activity in E. Coli cells. 'Normal' conditions refer to the voltage difference as measured by the thermopile upon addition of hydrogen peroxide to the cell, in the absence of sodium azide.

### Detailed description

The present invention provides a general and fast method for the detection of contaminants in samples of non-living material.

Preferably the sample in which the contaminant is to be determined does itself not comprise living cells, i.e. water, food material such as, but not limited to milk, flour, starch, sugar, etc, other non-food products such as soil, or certain medical preparations such as drugs or sera. Thus, according to a preferred embodiment of the invention, detection of any living cell is desired. This can also be the case for instance when effectiveness of sterilization of certain products needs to be confirmed.
It can however, also be envisaged that the samples to be investigated for the presence of bacterial contaminants do contain a certain fraction of living cells, such as plant cells. Preferably, the energy production mechanism in such cells in the sample should be blocked or inhibited (i.e. by working under non-light conditions).

The contaminants as used herein relate primarily to bacterial contaminants. The contamination by bacteria is an important concern for water and food products. In non-food products contamination by bacteria can also be problematic causing deterioration of quality due to the presence of impurities. Examples of contaminating bacteria are Listeria (pasteurized milk products), E. coli (drinking water), Legionella (heated fountain and shower water), or Salmonella (food products). However, it can be envisaged that for certain non-food products the absence of animal or human cells is a critical factor.

According to the present invention, contaminants of living cells, in a sample non-living material are detected by stimulating or blocking one or more enzymatic reactions within the contaminant, and measuring the associated energy change. In a preferred embodiment of the invention, a molecule is added to the sample which causes the uncoupling of the transport of protons from ATP production in living cells, so that ATP is no longer produced and heat is generated. According to this embodiment of the invention, heat will be generated if there are living cells present in the sample.

Molecules capable of uncoupling proton transport from ATP production are known in the art. The uncoupling protein (UCP) is a molecule present in the inner membrane of mitochondria. To date, at least three structurally related UCP molecules have been identified in humans (Cassard et al., Journal of Cell Biochemistry, 43, 1990; Fleury et al. in Nature Genetics, 1997, 15, 269; Boss O et al., FEBS Lett, May 12, 1997, 408(1), 39-42). US Patent 6,187,560 describes the uncoupling protein HNFCW60 and recombinant methods for their production.
Dinitrophenol (DNP) is a known poison which transports protons across the cell membrane thereby dissipating the proton gradient necessary for ATP production. Carbonyl Cyanide 3-Chlorophenylhydrazone (CCCF) is a proton ionophore which partially inhibits the pH gradient-activated Cl⁻ uptake and Cl⁻/Cl⁻ exchange activities in brush-border membrane vesicles. However, it can be envisaged that other molecules, capable of dissipation of the proton gradient across a cellular membrane, can be used in the context of the present invention.

Alternatively, it can be envisaged that molecules capable of stimulating the production of ATP by living cells such as bacteria which results in an increase of energy released, can also be used in the context of the present application.

Alternatively, according to the application, the presence of a living cell, more particularly a bacterial cell is detected by measuring the enzymatic degradation of hydrogen peroxide by catalase. This reaction occurs in all living cells and the detection of catalase activity in a sample is thus indicative of the presence of living cells. The degradation of hydrogen peroxide generates an energy release by the cell which can be measured.

According to the present invention, an increase in energy in the sample is measured by a microcalorimetric device, capable of measuring very small energy changes. In a preferred embodiment, the microcalorimetric device comprises a heat detection means. Most preferably the heat detection means is a differential heat detection means such as a thermopile. According to a preferred embodiment, a microcalorimeter as described in US patent 6,380,605 is used. In the latter, the thermopile measures the temperature difference between two recipients (or wells). The recipients hold samples in the microliter range. Because they are thermally well insulated from each other, any change in enthalpy between the two recipients is converted to a temperature change. The thermopile transduces this temperature difference in a voltage difference. However, it can be envisaged that other devices capable of measuring very small energy changes within a sample, such as devices based on heat radiation microcalorimetry, can be used for the methods of the present invention.

### Examples

### Example 1: Detection of living cells by uncoupling of oxidative phosphorylation in mitochondria

The uncoupling of oxidative phosphorylation in mitochondria can be achieved by the protonophore (H⁺ ionophore) carbonylcyanide-3-chlorophenylhydrazone (CCCF). This molecule has been shown to have a number of effects on cellular calcium. Inhibits secretion of hepatic lipase and partially inhibits the pH gradient-activated Cl⁻ uptake and Cl⁻/Cl⁻ exchange activities in brush-border membrane vesicles.

Hepatocyte cells were grown on 37 degrees Celcius with 5% CO2, Rinsed with Versene, then loosened with EDTA-trypsine and washed with PBS. Concentration was 1,88. 10e7 cells / ml or about 28 000 cells in a well.

The uncoupler CCCF was dissolved in DMSO to a stock concentration of 100 mM, the diluted to 10 mM again in DMSO, and a final working concentration of 400 µM was diluted in H₂O.

A sample of hepatocytes was introduced in the adjoining recipients (hereinafter referred to as top and bottom well) of a microcalorimeter. The thermopile, which is located between the two recipients allows the measurement of a minute temperature difference between the samples in each recipient by measuring a voltage difference. Sensitivity was determined to be 15mV/K (10.7V/W).
The following procedure was used:
- adding of 1.5 µl of hepatocyte solution was added to the top and bottom well of the microcalorimeter
- 0.5 µl of a 100 µM solution of uncoupler was added to the bottom well, while the same volume of the buffer solution was added to the top well
- At its maximum (reached after 10min), the voltage difference between the two wells was recorded. From this value, the temperature difference and the heat production difference could be determined.

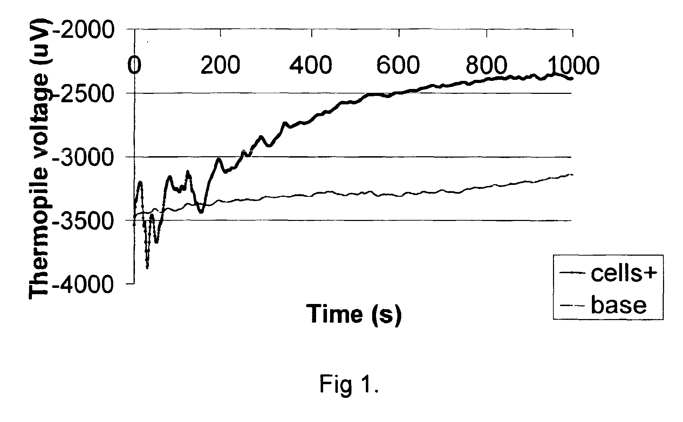

The result is demonstrated in Figure 1. The maximum voltage difference measures was 800µV. The temperature difference (corresponding to the Voltage difference divided by the sensitivity of the microcalorimeter) was determined to be 50 mK. Based on the number of cells added to each well, this corresponds to a heat production difference per cell of 3nW.

### Comparative example 2. Detection of bacterial cells based on catalase activity

The presence of living cells, including bacterial cells, in a sample can be detected by addition of hydrogen peroxide which will be degraded by catalase activity present in the cells, which causes heat production.

Escherichia coli bacteria were grown on 37° Celcius in Liquid Broth. Samples of bacteria are taken when growth is in the log fase and is estimated to be around 4.10e8 bacteria / ml, which corresponds to around 600 000 bacteria per well.

A 30 % (w/w) hydrogen peroxide solution from Sigma was used directly.

Measurements were performed using a microcalorimeter. The top well was provided with 1.5µl of the bacterial solution in LB, while the bottom well contained 1.5µl LB only.
When a steady baseline was obtained (10 min) 0.5µl of the hydrogen peroxide solution was added to each of the wells.

The maximum temperature difference was 400mK. The heat capacity, which is the sum of the heat capacity of water (4Kj/1K*volume) and that of silicon (2mj/K) goes from 8mJ/K before addition of hydrogen peroxide, to 10mJ/K after addition of the hydrogen peroxide. The total enthalpy change (which can be calculated for the used microcalorimeter based on the maximum temperature: Tmax=ΔH/2C) was determined to be 80mJ.

### Inhibition by sodium azide

In order to confirm that the heat production observed could be attributed to catalase activity, the same reaction was repeated, but in the presence of the catalase inhibitor sodium azide (NaN₃).

A solution of sodium azide was made to obtain a final concentration in the wells of 10 or 20mM. This was added to the top and bottom wells and the experiment as described above was repeated.

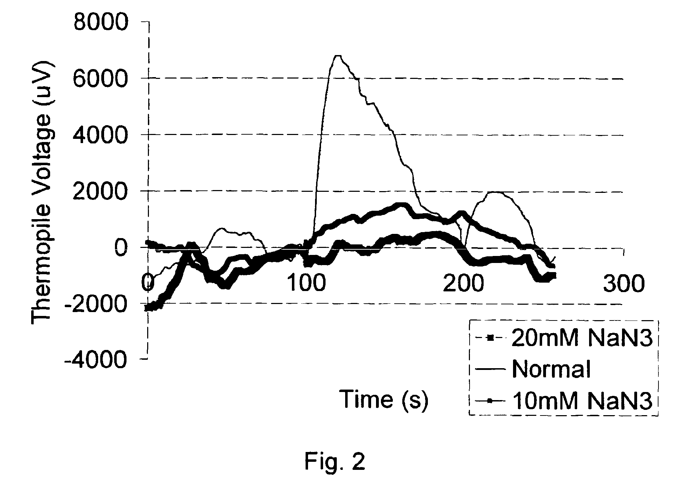

The effect of sodium azide on the catalase activity of E. coli in the sample is illustrated in Figure 2. It can be seen that in the presence of sodium azide, there is no detection of a voltage difference between the wells upon addition of hydrogen peroxide.

### Example 3: Detection of cellular contaminants in a food steam

samples of a food steam are taken and introduced into the microtiter-plate recipients of a microcalorimeter, based on differential heat detection means, capable of measuring a temperature difference between a reference and test recipient. A larger number of measurements increases the sensitivity, thus, for instance 96 samples are taken, and each sample is divided over four wells, filling a 384-well plate completely. An uncoupler, such as CCCF, is added to two test wells. The differential detection means identifies any temperature difference between the control and test wells (in duplo for each sample). If a temperature difference is noted between the control and the test wells, this means that living cells are present in the sample and is and indication of contamination.

## Claims

1. A method to detect the contamination of a sample with living cells, said method comprising:
- uncoupling electron transport system from ATP production in said living cells, by addition of an uncoupling agent to the sample
- detecting the energy production associated therewith in the sample by microcalorimetry
whereby the associated energy change is indicative of contamination of the sample.

2. The method of claim 1, wherein said energy production is measured by way of a heat conduction microcalorimetry.

3. The method of claim 1, wherein said energy production is measured by radiation based microcalorimetry.

4. The method of any one of claims 1 to 3, wherein said living cells are bacterial contaminants.

5. The method of any one of claims 1 to 3, wherein said living cells are human cells.

6. The method of claim 4, wherein said sample is a sample taken from water, food-material, or soil.

7. The method of claim 4, wherein said sample is a medical preparation, such as a drug or sera.

8. The method of any one of claims 1 to 7, wherein said uncoupling agent is the uncoupling protein (UCP), uncoupling protein HNFCW60 or a functional homologue of any one of these proteins, dinitrophenol (DNP), carbonyl cyanide 3-chlorophenylhydrazone (CCCF).

## Patentansprüche

1. Verfahren zum Nachweis der Kontamination einer Probe mit lebenden Zellen, wobei das Verfahren umfasst:
- Entkoppeln des Elektronentransportsystems von der ATP-Produktion in den lebenden Zellen durch Zugabe eines Entkoppelungsmittels zur Probe
- Nachweis der damit verbundenen Energieproduktion in der Probe durch Mikrocalorimetrie
wodurch die damit verbundene Energieänderung als Indikator einer Kontamination der Probe dient.

2. Verfahren nach Anspruch 1, wobei die Energieproduktion durch Wärmekonduktions-Mikrocalorimetrie bestimmt wird.

3. Verfahren nach Anspruch 1, wobei die Energieproduktion durch auf Bestrahlung basierender Mikrocalorimetrie bestimmt wird.

4. Verfahren nach einem der Ansprüche 1 bis 3, wobei die lebenden Zellen bakterielle Kontaminanten sind.

5. Verfahren nach einem der Ansprüche 1 bis 3, wobei die lebenden Zellen Humanzellen sind.

6. Verfahren nach Anspruch 4, wobei die Probe eine aus Wasser, Nahrungsmitteln oder Boden entnommene Probe ist.

7. Verfahren nach Anspruch 4, wobei die Probe eine medizinische Zubereitung ist, beispielsweise ein Wirkstoff oder ein Serum.

8. Verfahren nach einem der Ansprüche 1 bis 7, wobei das Entkoppelungsmittel das Entkoppelungsprotein (UCP), das Entkoppelungsprotein HNFCW60 oder ein funktionelles Homolog von einem dieser Proteine, Dinitrophenol (DNP), Carbonylcyanid 3-chlorphenylhydrazon (CCCF) ist.

## Revendications

1. Procédé pour détecter la contamination d'un échantillon avec des cellules vivantes, ledit procédé comprenant :
- le découplage du système de transport d'électrons provenant de la production d'ATP dans lesdites cellules vivantes, par l'ajout d'un agent de découplage à l'échantillon
- la détection de la production d'énergie associée à celui-ci dans l'échantillon par microcalorimétrie
par lequel le changement d'énergie associé est révélateur de la contamination de l'échantillon.

2. Procédé selon la revendication 1, dans lequel ladite production d'énergie est mesurée au moyen de la microcalorimétrie par conduction thermique.

3. Procédé selon la revendication 1, dans lequel ladite production d'énergie est mesurée par microcalorimétrie basée sur le rayonnement.

4. Procédé selon l'une quelconque des revendications 1 à 3, dans lequel lesdites cellules vivantes sont des contaminants bactériens.

5. Procédé selon l'une quelconque des revendications 1 à 3, dans lequel lesdites cellules vivantes sont des cellules humaines.

6. Procédé selon la revendication 4, dans lequel ledit échantillon est un échantillon provenant d'eau, de matière alimentaire, ou de terre.

7. Procédé selon la revendication 4, dans lequel ledit échantillon est une préparation médicale, telle qu'un médicament ou un sérum.

8. Procédé selon l'une quelconque des revendications 1 à 7, dans lequel ledit agent de découplage est la protéine de découplage (UCP), la protéine de découplage HNFCW60 ou un homologue fonctionnel de l'une quelconque de ces protéines, le dinitrophénol (DNP), le cyanure de carbonyle de la 3-chlorophénylhydrazone de (CCCF).
